# EUROPÄISCHE PATENTSCHRIFT

(11) **EP 2 178 883 B1**
(45) Veröffentlichungstag und Bekanntmachung des Hinweises auf die Patenterteilung: **24.04.2013**
(21) Anmeldenummer: 08785467.5
(22) Anmeldetag: 08.08.2008
(51) Int. Cl.: C07D 493/04, C07D 493/14, C09K 9/02, G02C 7/10

(54) **PHOTOCHROME BENZOPYRANO-BENZOPYRANE MIT WEITERER ANNELLIERUNG**
PHOTOCHROMIC BENZOPYRANO-BENZOPYRANS WITH FURTHER FUSING
BENZOPYRANO-BENZOPYRANES PHOTOCHROMES À ANNELLATION SUPPLÉMENTAIRE

(30) Priorität: 23.08.2007 DE 102007039994
(43) Veröffentlichungstag der Anmeldung: 28.04.2010
(73) Patentinhaber: Rodenstock GmbH, 80687 München (DE)
(72) Erfinder: MELZIG, Manfred, 82234 Wessling (DE); ROHLFING, Yven, 81547 München (DE); WEIGAND, Udo, 81247 München (DE)
(74) Vertreter: Müller-Boré & Partner Patentanwälte
(86) Internationale Anmeldenummer: PCT/EP2008/006575
(87) Internationale Veröffentlichungsnummer: WO 2009/024271

(56) Entgegenhaltungen:
- WO-A-00/02884
- WO-A-02/22594

## Beschreibung

Die vorliegende Erfindung betrifft photochrome Benzopyrano-benzopyrane mit weiterer Annellierung sowie deren Verwendung in Kunststoffen aller Art, insbesondere für ophthalmische Zwecke. Es handelt sich bei den erfindungsgemäßen Verbindungen um photochrome Benzopyran-Verbindungen, die auch als vom 9-Oxa-9,10-dihydrophenanthren abgeleitet angesehen werden können.

Seit langem sind verschiedene Farbstoffklassen bekannt, die bei Bestrahlung mit Licht bestimmter Wellenlängen, insbesondere Sonnenstrahlen, reversibel ihre Farbe wechseln. Dies geht darauf zurück, daß diese Farbstoffmoleküle durch Lichtenergie in einen angeregten Zustand übergehen, den sie bei Unterbrechung der Energiezufuhr wieder verlassen und in ihren Ausgangszustand zurückkehren. Zu diesen photochromen Farbstoffen gehören verschiedene Pyransysteme, die im Stand der Technik mit unterschiedlichen Grundsystemen und Substituenten bereits beschrieben wurden.

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen. Eine geeignete Verbindungsklasse von Pyranen sind zum Beispiel die 2,2-Diaryl-2H-naphtho[1,2-b]pyrane oder die 3,3-Diaryl-3H-naphtho[2,1-b]pyrane, die in angeregter Form verschiedene Färbungen, wie Gelb, Orange oder Rotorange, zeigen.

Als weitere Verbindungsklasse photochromer Verbindungen sind höher annellierte Pyrane von Interesse, die aufgrund ihres größeren Ringsystems längerwellig absorbieren und rote, violette und blaue Farbtöne ergeben. Diese können entweder von den 2H-Naphtho[1,2-b]pyranen oder den 3H-Naphtho[2,1-b]pyranen abgeleitete Systeme sein, die durch Annellierung an der f-Seite aus den jeweiligen Naphthopyran-Systemen hervorgehen.

Diarylchromene, insbesondere Naphthopyrane oder heterozyklisch annellierte Benzopyrane, die in 6-Stellung des Benzopyrans mit einem Phenylring oder allgemeiner einem aromatischen oder heteroaromatischen Ring substituiert sind, welcher zusätzlich über die 5-Stellung des Benzopyrans über mindestens ein Kohlenstoffatom, Sauerstoffatom oder Stickstoffatom verbrückt ist, sind derzeit die vielversprechendsten photochromen Verbindungen.

Wird diese Verbrückung nur über ein Atom erzeugt, so ergibt sich ein an das Benzopyran annellierter Fünfring, Beispiele finden sich für ein Kohlenstoffatom in US 5,645,767, US 5,723,072 sowie US 5,955,520 und für ein Sauerstoffatom in US 6,018,059.

In US 5,723,072 kann zusätzlich an diesem Grundsystem ein un-, mono- oder disubstituierter heterozyklischer Ring an der g-, h-, i-, n-, o- oder p-Seite des Indenonaphthopyrans annelliert sein. Es werden demzufolge Indeno[1,2-f]naphtho[1,2-b]pyrane mit einer sehr großen Variationsbreite an möglichen Substituenten offenbart.

In WO 96/14596, WO 99/15518, US 5,645,767, WO 98/32037 und US 5,698,141 werden vom 2H-Naphtho[1,2-b]pyran abgeleitete photochrome indenoannellierte Naphthopyran-Farbstoffe, die sie enthaltenden Zusammensetzungen sowie ein Verfahren zu ihrer Herstellung offenbart. In US 5,698,141 kann zusätzlich an diesem Grundsystem ein un-, mono- oder disubstituierter heterozyklischer Ring an der g-, h-, i-, n-, o- oder p-Seite des Indenonaphthopyrans annelliert sein. Von der jeweils sehr umfangreichen Substituentenliste sind auch ganz spezielle Spiro-Verbindungen umfaßt, und zwar solche Systeme mit einer spiroheterozyklischen Gruppe, worin einschließlich des Spiroatoms an der 13-Position des Grundsystems ein 5- bis 8-gliedriger Ring, der stets zwei Sauerstoffatome enthält, vorhanden ist. Eine weitere Ausführungsform des Spiroringes findet sich in der japanischen Anmeldung 344762/2000.

Wird diese Verbindung über zwei Atome erzeugt, so ergibt sich ein annellierter Sechsring mit allein für C, O und N vielfältigen Möglichkeiten. Verbindungen mit C=O und N-R (Lactam-Brücke) werden in US 6,379,591 beschrieben. Verbindungen mit einer unsubstituierten CH₂-CH₂-Brücke sowie einem annellierten Heterocyclus in 7,8-Stellung des zugrundeliegenden Benzopyrans sind in US 6,426,023 offenbart.

US 6,506,538 beschreibt die carbocyclischen Analogverbindungen, bei denen die H-Atome in der Brücke durch OH, (C₁ - C₆) Alkoxy oder zwei H-Atome an einem C-Atom durch =O ersetzt sein können. Alternativ kann auch eines der Kohlenstoffatome in der zweigliedrigen Brücke durch Sauerstoff ersetzt sein. Diese Verbindungen sind neben weiteren in WO 00/02884 beschrieben.

Wird diese Verbindung durch drei Atome erzeugt, ergibt sich ein annellierter 7-Ring mit sehr vielen Variationsmöglichkeiten durch Einfügen von Heteroatomen. Verbindungen mit einer CH₂-CH₂-CH₂-Brücke sind in US 6,558,583 beschrieben. Auch hier können die H-Atome in der Brücke durch OH, (C₁ - C₆)-Alkyl oder (C₁ - C₆)-Alkoxy oder zwei Wasserstoffatome an einem C-Atom durch =O ersetzt sein. Sie absorbieren bei gleichem Substitutionsmuster kürzerwellig als die annellierten 6-Ringe.

US 2004/0094753 beschreibt sowohl Verbindungen mit 2- wie mit 3-atomiger Brücke. Die zweiatomige (Kohlenstoff-)Brücke ist dabei zusätzlich mit einem Carbo- bzw. Heterocyclus annelliert. Die dreiatomige Brücke enthält drei C-Atome oder zwei C-Atome und ein O-Atom ohne zusätzliche Annellierung. Beide Ringe können vielfältige Substituenten tragen.

Die verschiedenen, im Stand der Technik verfügbaren photochromen Farbstoffe haben jedoch Nachteile, die bei der Verwendung in Sonnenschutzgläsern den Tragekomfort des Brillenträgers wesentlich beeinträchtigen. Zum einen weisen die Farbstoffe eine nicht ausreichend langwellige Absorption im angeregten wie im nicht angeregten Zustand auf. Zum anderen liegt häufig eine zu hohe Temperaturempfindlichkeit der Eindunkelung vor, wobei gleichzeitig eine zu langsame Aufhellung eintreten kann. Darüber hinaus besitzen die im Stand der Technik verfügbaren Farbstoffe oft eine ungenügende Lebensdauer und erlauben damit nur eine geringe Haltbarkeit der Sonnenschutzgläser. Letzteres macht sich in schnell nachlassender Leistung und/oder starker Vergilbung bemerkbar.

Der vorliegenden Erfindung liegt daher die Aufgabe zugrunde, eine Klasse photochromer Verbindung bereitzustellen, die - beschränkt auf Systeme mit einer zweiatomigen Brücke - deutlich verbesserte Eigenschaften gegenüber den im Stand der Technik beschriebenen Strukturen besitzen sollen. Diese sind in der Kombination von langwelligem Absorptionsmaximum der geschlossenen Form mit steiler Kante zum sichtbaren Wellenlängenbereich, hoher Eindunkelungsleistung, sehr schneller Aufhellreaktion und sehr guter Lichtbeständigkeit zu finden.

Diese Aufgabe wird durch die in den Ansprüchen gekennzeichneten Gegenstände gelöst.

Insbesondere werden photochrome Benzopyrano-benzopyrane mit weiterer Annellierung mit der allgemeinen Formel (I) bereitgestellt: 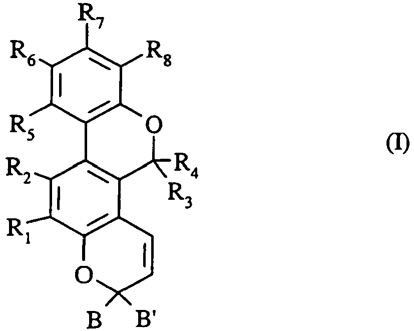 worin
mindestens einer der Reste R₆, R₇ oder R₈ die folgende Einheit (A) darstellt, 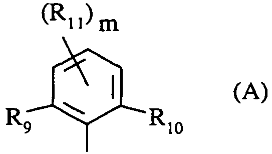 mit der Maßgabe, daß R₉ oder R₁₀ zusammen mit einem zur Kopplungsstelle ortho-ständigen Rest R₅, R₆, R₇ oder R₈ eine Verbrückung bildet, oder R₉ und R₁₀ im Falle einer Kopplung der vorstehenden Einheit (A) über R₆ jeweils zusammen mit beiden zur Kopplungsstelle ortho-ständigen Resten R₅ und R₇ bzw. im Falle einer Kopplung der vorstehenden Einheit (A) über R₇ mit beiden zur Kopplungsstelle ortho-ständigen Resten R₆ und R₈ zwei Verbrückungen bilden, wobei die Verbrückung über die Reste R₉ bzw. R₁₀ jeweils eine solche ist, ausgewählt aus der Gruppe, bestehend aus -CR₁₂R₁₃-, -O-, -S-, -N(Ph)-, -N(C₁-C₆ Alkyl)-, -O-CR₁₂R₁₃-, -S-CR₁₂R₁₃-, -CR₁₂R₁₃-CR₁₂R₁₃-, -CR₁₄=CR₁₅- oder -CR₁₅=N-,
oder
worin der Rest R₅ mit dem Rest R₆ oder der Rest R₆ mit dem Rest R₇ oder der Rest R₇ mit dem Rest R₈ oder der Rest R₅ mit dem Rest R₆ und gleichzeitig der Rest R₇ mit dem Rest R₈ einen un-, mono- oder disubstituierten Benzo-, Pyrido-, Furo- oder Thienoring darstellen, wobei die Substituenten jeweils ausgewählt sein können aus der Gruppe α, bestehend aus einem Wasserstoffatom, einem (C₁ - C₆)-Alkylrest, einem (C₁ - C₆)-Thioalkylrest, einem (C₃ - C₇)-Cycloalkylrest, der ein oder mehrere Heteroatome, wie beispielsweise O oder S, aufweisen kann, einem (C₁ - C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α ausgewählt sein können;
und worin
die Reste R₁ und R₂ jeweils unabhängig voneinander einen Substituenten darstellen, ausgewählt aus der vorstehenden Gruppe α; oder
die Reste R₁ und R₂ eine an den aromatischen Ring gebundene -A-(CH₂)ₖ-D-Gruppe oder -A-(C(CH₃)₂)ₖ-D- Gruppe mit k = 1 oder 2 bilden, wobei A und D unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann; oder
die Reste R₁ und R₂ einen unsubstituierten, mono- oder disubstituierten Benzo- oder Pyridoring darstellen, dessen Substituenten aus der Gruppe α ausgewählt sein können;
die Reste R₃ und R₄ entweder jeweils unabhängig voneinander aus der Gruppe α ausgewählt sind, oder die Reste R₃ und R₄ zusammen unter Einbeziehung des Spiro-Kohlenstoffatoms einen 3- bis 8-gliedrigen carbo- oder heteromonozyklischen Spiro-Ring, der gegebenenfalls einen oder mehrere Substituenten aus der Gruppe α trägt, bilden, an den ein bis drei aromatische oder heteroaromatische Ringsysteme annelliert sein können, wobei das Ringsystem unabhängig voneinander aus der Gruppe β, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten aus der Grupppe α substituiert sein können, ausgewählt ist, wobei zwei benachbarte annellierte Ringsysteme auch durch eine ortho,ortho'-Brücke miteinander verknüpft sein können, oder die Reste R₃ und R₄ zusammen unter Einbeziehung des Spiro-Kohlenstoffatoms einen 7- bis 12-gliedrigen carbobizyklischen Spiro-Ring oder einen 7- bis 12-gliedrigen carbo-trizyklischen Spiro-Ring bilden, die jeweils gegebenenfalls ein oder mehrere Substituenten aus der Gruppe α tragen können,
die Reste R₅, R₆, R₇, R₈, R₉ und R₁₀, sofern sie nicht eine Verbrückung bilden, sowie der Rest R₁₁ jeweils unabhängig voneinander einen Substituenten darstellen, ausgewählt aus der Gruppe α, oder
R₅ und R₆ im Falle einer Kopplung der vorstehenden Einheit (A) über R₈ einen annellierten, unsubstituierten, mono- oder disubstituierten Benzo-, Naphtho- oder Pyridoring darstellen, dessen Substituenten aus der Gruppe α ausgewählt sein können;
oder zwei zueinander orthoständige Reste R₁₁ eine an den aromatischen Ring gebundene -A-(CH₂)ₖ-D- Gruppe oder -A-(C(CH₃)₂)ₖ-D- Gruppe mit k = 1 oder 2, wie vorstehend definiert, bilden, oder zwei zueinander orthoständige Reste R₁₁ einen unsubstituierten, mono- oder disubstituierten Benzo- oder Pyridoring darstellen, dessen Substituenten aus der Gruppe α ausgewählt sein können;
die Reste R₁₂, R₁₃, R₁₄ und R₁₅ jeweils unabhängig voneinander einen Substituenten darstellen, ausgewählt aus der Gruppe α,
oder R₁₂ und R₁₃ zusammen mit einem zur Kopplungsstelle meta-ständigen Rest R₁₁ einen annellierten, unsubstituierten, mono- oder disubstituierten Benzo-, Naphtho- oder Pyridoring darstellen, dessen Substituenten aus der Gruppe α ausgewählt sein können, wobei dies bei einer Verbrückung mittels der Gruppen -O-CR₁₂R₁₃- bzw. -S-CR₁₂R₁₃- nur möglich ist, wenn CR₁₂R₁₃ direkt an den Phenylring, der R₁₁ trägt, gebunden ist, oder
im Falle einer Kopplung der vorstehenden Einheit (A) über R₇ und einer Verbrückung über R₆ die Reste R₁₂ und R₁₃ zusammen mit R₅ bzw. im Falle einer Kopplung der vorstehenden Einheit (A) über R₈ und einer Verbrückung über R₇ die Reste R₁₂ und R₁₃ zusammen mit R₆ bzw. im Falle einer Kopplung der vorstehenden Einheit (A) über R₆ und einer Verbrückung über R₇ die Reste R₁₂ und R₁₃ zusammen mit R₈ einen annellierten, unsubstituierten, mono- oder disubstituierten Benzo-, Naphtho- oder Pyridoring darstellen, dessen Substituenten aus der Gruppe α ausgewählt sein können, oder
oder R₁₄ und R₁₅ einen annellierten, unsubstituierten, mono- oder disubstituierten Benzo-, Naphtho- oder Pyridoring darstellen, dessen Substituenten aus der Gruppe α ausgewählt sein können;
m 0, 1, 2 oder 3 ist und
B und B' unabhängig voneinander aus einer der folgenden Gruppen a), b) oder c) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist;
   wobei die Substituenten der Aryl- oder Heteroarylreste in a) und b) solche sind, ausgewählt aus der vorstehend definierten Gruppe α oder der Gruppe χ, bestehend aus Hydroxy, am Phenylring un-, mono- oder disubstituiertem 2-Phenylethenyl, am Phenylring un-, mono- oder disubstituiertem (Phenylimino)methylen, am Phenylring un-, mono- oder disubstituiertem (Phenylmethylen)imino, Amino, Mono-(C₁ - C₆)-alkylamino, Di-(C₁ - C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,flazepinyl, wobei der oder die Substituenten unabhängig voneinander wiederum aus (C₁ - C₆)-Alkyl, (C₁ - C₆)-Alkoxy, Brom, Chlor oder Fluor ausgewählt sein können;
   oder wobei zwei direkt benachbarte Substituenten eine Y-(CX₂)ₚ-Z-Gruppierung darstellen, wobei p = 1, 2 oder 3 ist, X Wasserstoff, CH₃ oder C₆H₅ sein kann und Y und Z unabhängig voneinander Sauerstoff, Schwefel, N-(C₁ - C₆)-Alkyl, N-C₆H₅, CH₂, C(CH₃)₂ oder C(C₆H₅)₂ sein können, wobei zwei oder mehrere benachbarte Kohlenstoffatome dieser Y-(CX₂)ₚ-Z-Gruppierung jeweils unabhängig voneinander auch Teil eines daran annellierten Benzo-Ringsystems sein können, welches jeweils wiederum einen oder mehrere Substituenten, ausgewählt aus der Gruppe α oder der Gruppe χ, aufweisen kann;
   oder
c) B und B' zusammen mit dem benachbarten Kohlenstoffatom des Pyran-Ringes einen un-, mono- oder disubstituierten 9,10-Dihydroanthracen-, Fluoren-, Thioxanthen-, Xanthen-, Benzo[b]fluoren-, 5H-Dibenzo[a,d]cyclohepten oder Dibenzosuberon-Rest oder einen gesättigten Kohlenwasserstoffrest, der (C₃ - C₁₂)-spiro-monozyklisch, (C₇ - C₁₂)-spiro-bizyklisch bzw. (C₇ - C₁₂)-spiro-trizyklisch ist, bilden, wobei die Substituenten der ungesättigten Cyclen unabhängig voneinander aus der Gruppe α oder der Gruppe χ ausgewählt sein können.

Die erfindungsgemäßen, von Benzopyrano-benzopyranen abgeleiteten photochromen Verbindungen weisen im Vergleich zu derzeit im Stand der Technik verfügbaren Systemen mit einer zweiatomigen Brücke ein deutlich verbessertes Eigenschaftsprofil, insbesondere eine verbesserte Kombination von sehr guter Lebensdauer sowie schneller Aufhellungsgeschwindigkeit auf. Darüberhinaus zeigen die erfindungsgemäßen Verbindungen gegenüber solchen mit einer einatomigen Brücke neben einer schnelleren Aufhellungsgeschwindigkeit auch eine geringere Solvatochromie. Die erfindungsgemäßen Verbindungen zeigen eine ausgewogene Balance von langwelligem Absorptionsmaximum, hoher Eindunkelungsleistung, sehr schneller Aufhellreaktion und sehr guter Lichtbeständigkeit.

Bevorzugte photochrome Benzopyrano-benzopyrane mit weiterer Annellierung gemäß der vorliegenden Erfindung weisen die nachfolgende allgemeine Formel (II) auf: 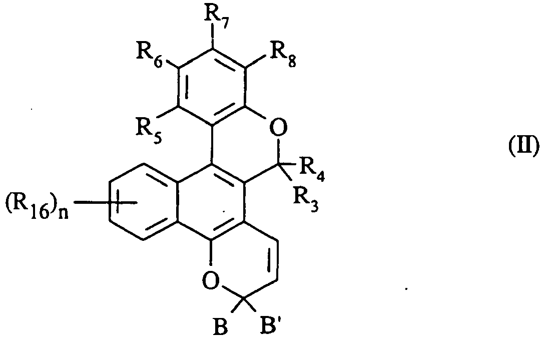 worin B, B', R₃, R₄, R₅, R₆, R₇ und R₈ wie vorgenannt definiert sind, R₁₆ aus der Gruppe α ausgewählt ist und n 0, 1, 2, 3 oder 4 ist.

Die Reste R₃ und R₄ können zusammen unter Einbeziehung des Spiro-Kohlenstoffatoms einen 3- bis 8-gliedrigen carbo- oder heterozyklischen Ring bilden, an den ein bis drei aromatische oder heteroaromatische Ringsysteme annelliert sein können, wobei das Ringsystem aus der Gruppe β, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten aus der Grupppe α substituiert sein können, ausgewählt ist. Dabei können auch zwei benachbarte annellierte Ringsysteme durch eine ortho,ortho'-Brücke, vorzugsweise eine Ethylen- oder eine 1,2-Ethendiyl-Brücke miteinander verknüpft sein, so daß beispielsweise im letzteren Fall folgende Struktureinheit vorliegt:

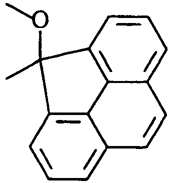

In einer bevorzugten Ausführungsform sind jedoch die Reste R₃ und R₄ jeweils unabhängig voneinander aus der Gruppe α ausgewählt.

Wenn B bzw. B' für einen gesättigten Kohlenwasserstoffrest steht, der C₃ - C₁₂ spiro-monozyklisch, C₇ - C₁₂ spiro-bizyklisch oder C₇ - C₁₂ spiro-trizyklisch ist, so werden unter C₃ - C₁₂ spiro-monozyklisch ein dem Fachmann geläufiger 3-gliedriger bis 12-gliedriger Ring verstanden. Auch C₇-C₁₂ spiro-bizyklische Systeme sind einem Fachmann wohlbekannt. Beispielhaft kann hier wiederum Norbornan, Norbornen, 2,5-Norbornadien, Norcaran und Pinan genannt werden. Ein beispielhaftes C₇ - C₁₂ spiro-trizyklisches System ist Adamantan.

In einer weiteren bevorzugten Ausführungsform sind die Reste B und B' unabhängig voneinander aus der Gruppe a), wie vorstehend definiert, ausgewählt.

Die Substituenten der Gruppe χ, welche Stickstoffatome aufweisen bzw. Amingruppen tragen, sind über diese an den Phenyl, Naphthyl- bzw. Phenanthrylrest der Gruppe a) gebunden.

Wenn bezüglich der Substituenten der Gruppe χ, welche an den Phenyl, Naphthyl- bzw. Phenanthrylrest der Gruppe a) für die Reste B bzw. B' gebunden sein können, zwei oder mehrere benachbarte Kohlenstoffatome dieser Y-(CX₂)ₚ-Z-Gruppierung jeweils unabhängig voneinander Teil eines daran annellierten Benzo-Ringsystems sein können, so bedeutet dies, dass dann die beiden Methylenkohlenstoffatome (-CH₂ - CH₂-) Teil eines annellierten Ringsystems werden. Wenn beispielsweise zwei oder drei Benzoringe annelliert sind, so können beispielsweise hier dann folgende Struktureinheiten vorliegen, wie nachstehend angeführt.

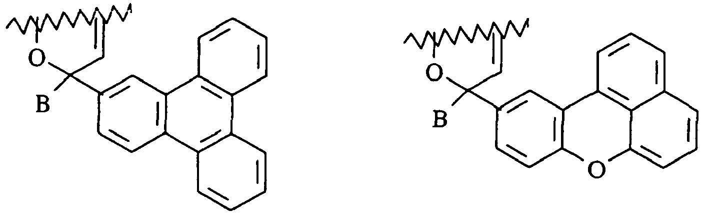

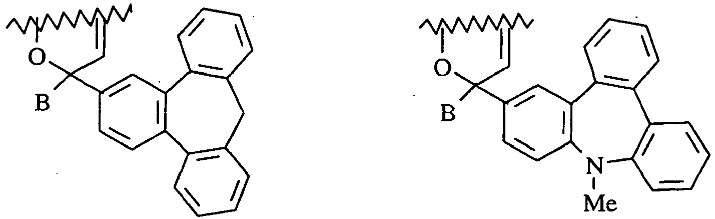

Selbstverständlich kann aber auch nur ein, über zwei benachbarte Kohlenstoffatome dieser Y-(CX₂)ₚ-Z-Gruppierung annellierter Benzoring vorliegen.

Bevorzugte photochrome Benzopyrano-benzopyrane-Derivate gemäß der vorliegenden Erfindung weisen die nachfolgenden allgemeinen Formeln (III) bzw. (IV) auf: 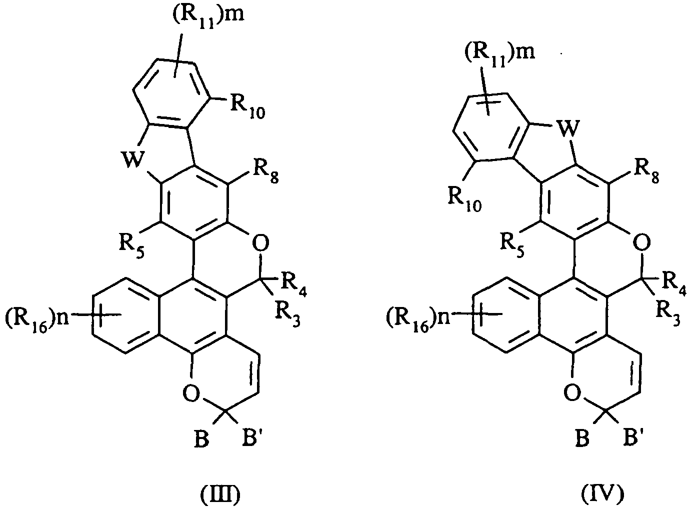 worin B, B', R₃, R₄, R₅, R₈, R₁₀ und R₁₆ wie vorgenannt definiert sind und W die vorstehend angeführte Verbrückung symbolisiert (die Brücke wird in diesen beiden Fällen von den Resten R₆ und R₉ gebildet und ist wie vorgenannt definiert, d.h. ausgewählt aus der Gruppe, bestehend aus -CR₁₂R₁₃-, -O-, -S-, -N(Ph)-, -N(C₁-C₆ Alkyl)-, -O-CR₁₂R₁₃-, -S-CR₁₂R₁₃-, -CR₁₂R₁₃-CR₁₂R₁₃-, -CR₁₄=CR₁₅- oder -CR₁₅=N-).

Eine weitere besonders bevorzugte Ausführungsform gemäß der vorliegenden Erfindung stellen Verbindungen gemäß den nachfolgenden Formeln (V), (VI) und (VII) dar: 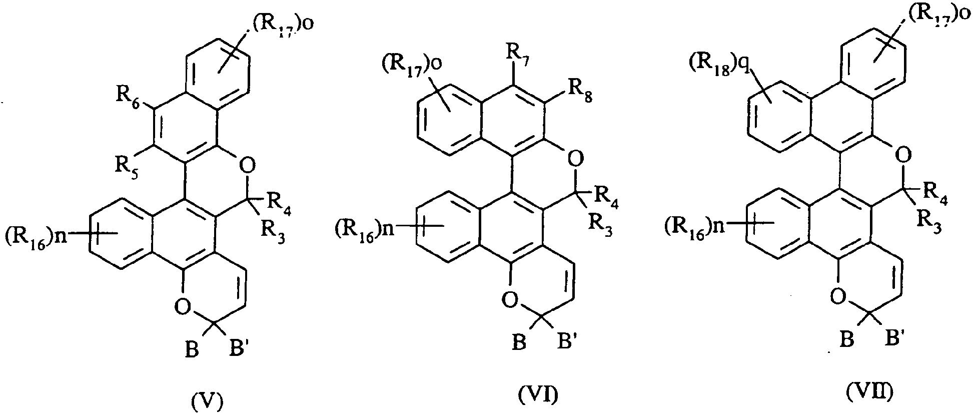 worin B, B', R₃, R₄, R₅, R₆, R₇, R₈, R₁₆ und n wie vorgenannt definiert sind, R₁₇ sowie R₁₈ aus der Gruppe α ausgewählt sind und o und q unabhängig voneinander 0, 1, 2, 3 oder 4 sind.

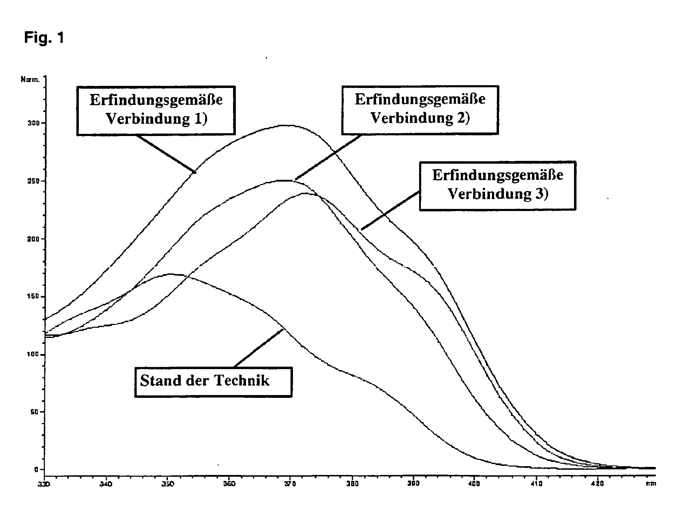

Im Vergleich zum Stand der Technik, d.h. WO 00/02884, weisen die erfindungsgemäßen Verbindungen - bei sonst gleichen Substituenten B, B', R₃ und R₄ - eine deutlich längerwellige Absorptionsbande sowohl im nicht angeregten als auch im angeregten Zustand auf. Eine längerwellige Absorption im nicht angeregten Zustand hat beim Einbringen der photochromen Farbstoffe z.B. in Kunststoff-Brillengläsern zwei wichtige Vorteile. Zum einen reagieren die erfindungsgemäßen Verbindungen auch dann, wenn bei ungünstigen atmosphärischen Bedingungen nur sehr langwelliges UV-Sonnenlicht (ab 380 nm) einfällt. Aus Figur 1 ist ersichtlich, dass die erfindungsgemäßen Verbindungen in der nicht angeregten Form bei Wellenlängen von größer als 370 nm deutlich intensiver absorbieren als im Vergleich zu Verbindungen des Standes der Technik. Dadurch zeigen die erfindungsgemäßen photochromen Verbindungen auch bei ungünstigen Bedingungen eine sehr gute Eindunklungsleistung. Zum anderen wird dadurch automatisch ein vollständiger UV-Schutz bis 400 nm erreicht, da die erfindungsgemäßen Verbindungen das einfallende UV-Licht komplett absorbieren. Ein Zusatz von UV-Absorbern bei der Herstellung von Sonnenschutzgläsern erübrigt sich. Dies ist ein großer Vorteil, da zugemischte UV-Absorber immer auch einen Teil des Anregungslichts absorbieren, so daß Gläser mit UV-Absorber immer weniger stark eindunkeln als ohne.

Die Struktur der in der Figur 1 dargestellten erfindungsgemäßen Verbindungen sowie deren längstwellige Absorptionsmaxima in der angeregten Form sind aus der folgenden Tabelle 1 ersichtlich (im Vergleich zum Stand der Technik aus WO 00/02884):

**Tabelle 1: Längstwellige Absorptionsmaxima im angeregten Zustand**

| | | | | | | | | | | | |
|---|---|---|---|---|---|---|---|---|---|---|---|
| 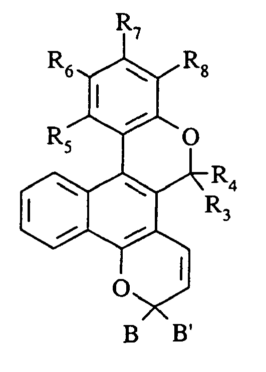 | | | | | | | | 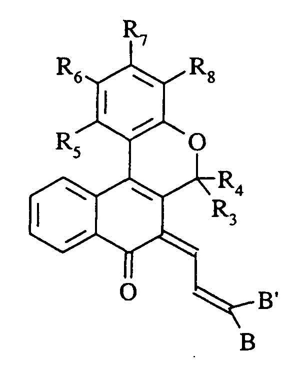 | | | |
| nicht angeregt (farblos) | | | | | | | | angeregt (farbig) | | | |

| A) Stand der Technik (WO 00/02884) | | | | | | | | | | | |
|---|---|---|---|---|---|---|---|---|---|---|---|
| R₃ | R₄ | R₅ | R₆ | R₇ | R₈ | B | B' | | | λₘₐₓ (angeregt) | |
| H | H | H | H | H | H | Phenyl | 4-(N-Morpholinyl)phenyl | | | 565 nm | |

| B) Erfindungsgemäße Verbindungen | | | | | | | | | | | |
|---|---|---|---|---|---|---|---|---|---|---|---|
| Verb. | R₃ | R₄ | R₅ | R₆ | | R₇ | R₈ | B | B' | | λₘₐₓ (angeregt) |
| 1) | H | H | H | H | | Benzo | | Phenyl | 4-(N-Morpholinyl)phenyl | | 590 nm |
| 2) | H | H | Benzo | | | H | H | Phenyl | 4-(N-Morpholinyl)phenyl | | 585 nm |
| 3) | H | H | H | Benzofuro (gemäß Formel (III)) | | | H | Phenyl | 4-(N-Morpholinyl)phenyl | | 585 nm |

Zur Messung der Eigenschaften der erfindungsgemäßen photochromen Farbstoffe sowie der Verbindung aus dem Stand der Technik (s.o.) wurden jeweils 500 ppm des Farbstoffes in einer Acrylatmonomer-Matrix gelöst und nach Zusatz eines Polymerisations-Initiators mit Hilfe eines Temperaturprogrammes thermisch polymerisiert. Die Transmisionseigenschaften der so hergestellten Kunststoffgläser (Dicke 2mm) wurden anschließend nach DIN EN ISO 8980-3 vermessen.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Benzopyrano-benzopyrane-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen photochromen Benzopyrano-benzopyrane beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Benzopyrano-benzopyrane durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen Benzopyrano-benzopyrane beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

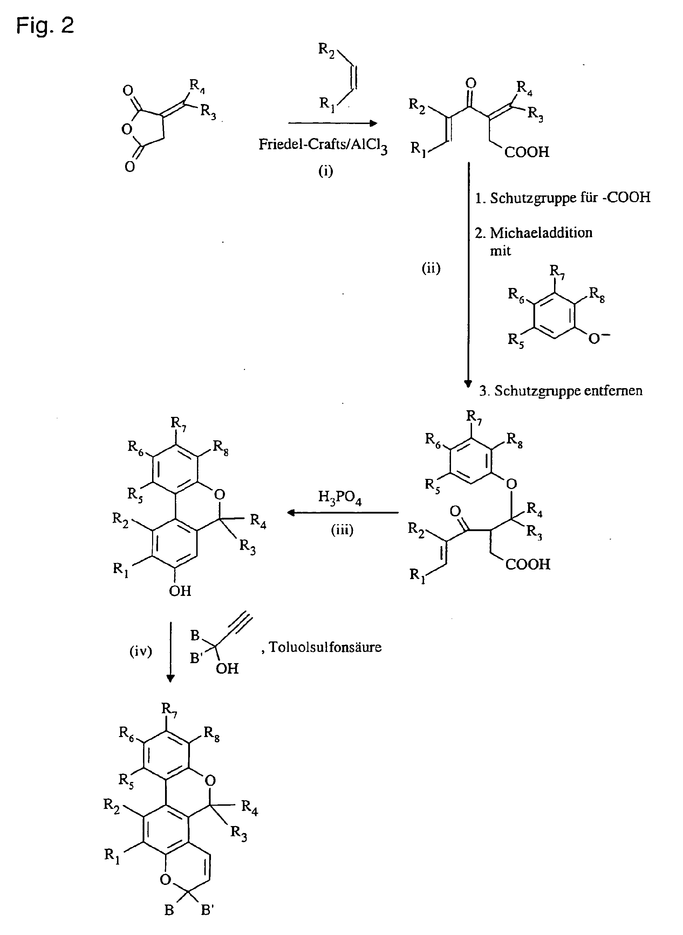

Die erfindungsgemäßen photochromen Verbindungen lassen sich gemäß folgendem beispielhaften Syntheseschema, wie in Figur 2 aufgezeigt, herstellen.

Geeignet substituierte Methylidensuccinanhydride werden in einem ersten Schritt einer Friedel-Crafts-Reaktion mit geeignet substituierten 1,2-Ethylenen unterworfen (Schritt (i)). Die -COOH Gruppe des daraus resultierenden Intermediats wird anschließend geschützt und dieses Intermediat einer Michael-Addition mit entsprechend substituierten Phenolatderivaten unterworfen (Schritt (ii)). Nach Entfernung der Carbonsäure-Schutzgruppe werden via intramolekularer Cyclisierung mittels Phosphorsäure entsprechend substituierte 9-Oxa-9,10-dihydrophenanthren-Derivate gebildet (Schritt (iii)). Anschließend werden diese substituierten 9-Oxa-9,10-dihydrophenanthren-Derivate mit geeignet substituierten 2-Propin-1-ol-Derivaten gemäß Schritt (iv) zu den erfindungsgemäßen Verbindungen umgesetzt.

## Patentansprüche

1. Photochrome Benzopyrano-benzopyrane mit weiterer Annellierung mit der allgemeinen Formel (I) bereitgestellt: 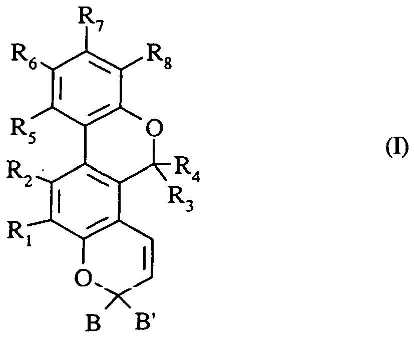 wobei mindestens einer der Reste R₆, R₇ oder R₈ folgende Einheit (A) darstellt, 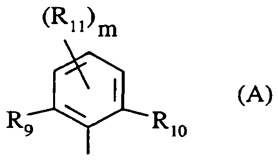 mit der Maßgabe, daß R₉ oder R₁₀ zusammen mit einem zur Kopplungsstelle ortho-ständigen Rest R₅, R₆, R₇ oder R₈ eine Verbrückung bildet, oder R₉ und R₁₀ im Falle einer Kopplung der vorstehenden Einheit (A) über R₆ jeweils zusammen mit beiden zur Kopplungsstelle ortho-ständigen Resten R₅ und R₇ bzw. im Falle einer Kopplung der vorstehenden Einheit (A) über R₇ mit beiden zur Kopplungsstelle ortho-ständigen Resten R₆ und R₈ zwei Verbrückungen bilden, wobei die Verbrückung über die Reste R₉ bzw. R₁₀ jeweils eine solche ist, ausgewählt aus der Gruppe, bestehend aus -CR₁₂R₁₃-, -O-, -S-, -N(Ph)-, -N(C₁-C₆ Alkyl)-, -O-CR₁₂R₁₃-, -S-CR₁₂R₁₃-, -CR₁₂R₁₃-CR₁₂R₁₃-, -CR₁₄=CR₁₅- oder -CR₁₅=N-,
oder
worin der Rest R₅ mit dem Rest R₆ oder der Rest R₆ mit dem Rest R₇ oder der Rest R₇ mit dem Rest R₈ oder der Rest R₅ mit dem Rest R₆ und gleichzeitig der Rest R₇ mit dem Rest R₈ einen un-, mono- oder disubstituierten Benzo-, Pyrido-, Furo- oder Thienoring darstellen, wobei die Substituenten jeweils ausgewählt sein können aus der Gruppe α, bestehend aus einem Wasserstoffatom, einem (C₁ - C₆)-Alkylrest, einem (C₁ - C₆)-Thioalkylrest, einem (C₃ - C₇)-Cycloalkylrest, der ein oder mehrere Heteroatome, wie beispielsweise O oder S, aufweisen kann, einem (C₁ - C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α ausgewählt sein können;
und worin
die Reste R₁ und R₂ jeweils unabhängig voneinander einen Substituenten darstellen, ausgewählt aus der vorstehenden Gruppe α; oder
die Reste R₁ und R₂ eine an den aromatischen Ring gebundene -A-(CH₂)ₖ-D-Gruppe oder -A-(C(CH₃)₂)ₖ-D- Gruppe mit k = 1 oder 2 bilden, wobei A und D unabhängig voneinander aus Sauerstoff, Schwefel, CH₂, C(CH₃)₂ oder C(C₆H₅)₂ ausgewählt sind, und wobei an diese -A-(CH₂)ₖ-D- Gruppe wiederum ein Benzoring annelliert sein kann; oder
die Reste R₁ und R₂ einen unsubstituierten, mono- oder disubstituierten Benzo- oder Pyridoring darstellen, dessen Substituenten aus der Gruppe α ausgewählt sein können;
die Reste R₃ und R₄ entweder jeweils unabhängig voneinander aus der Gruppe α ausgewählt sind, oder die Reste R₃ und R₄ zusammen unter Einbeziehung des Spiro-Kohlenstoffatoms einen 3- bis 8-gliedrigen carbo- oder heteromonozyklischen Spiro-Ring, der gegebenenfalls einen oder mehrere Substituenten aus der Gruppe α trägt, bilden, an den ein bis drei aromatische oder heteroaromatische Ringsysteme annelliert sein können, wobei das Ringsystem unabhängig voneinander aus der Gruppe β, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten aus der Grupppe α substituiert sein können, ausgewählt ist, wobei zwei benachbarte annellierte Ringsysteme auch durch eine ortho,ortho'-Brücke miteinander verknüpft sein können, oder die Reste R₃ und R₄ zusammen unter Einbeziehung des Spiro-Kohlenstoffatoms einen 7- bis 12-gliedrigen carbobizyklischen Spiro-Ring oder einen 7- bis 12-gliedrigen carbo-trizyklischen Spiro-Ring bilden, die jeweils gegebenenfalls ein oder mehrere Substituenten aus der Gruppe α tragen können,
die Reste R₅, R₆, R₇, R₈, R₉ und R₁₀, sofern sie nicht eine Verbrückung bilden, sowie der Rest R₁₁ jeweils unabhängig voneinander einen Substituenten darstellen, ausgewählt aus der Gruppe α, oder
R₅ und R₆ im Falle einer Kopplung der vorstehenden Einheit (A) über R₈ einen annellierten, unsubstituierten, mono- oder disubstituierten Benzo-, Naphtho- oder Pyridoring darstellen, dessen Substituenten aus der Gruppe α ausgewählt sein können;
oder zwei zueinander orthoständige Reste R₁₁ eine an den aromatischen Ring gebundene -A-(CH₂)ₖ-D- Gruppe oder -A-(C(CH₃)₂)ₖ-D- Gruppe mit k = 1 oder 2, wie vorstehend definiert, bilden, oder zwei zueinander orthoständige Reste R₁₁ einen unsubstituierten, mono- oder disubstituierten Benzo- oder Pyridoring darstellen, dessen Substituenten aus der Gruppe α ausgewählt sein können;
die Reste R₁₂, R₁₃, R₁₄ und R₁₅ jeweils unabhängig voneinander einen Substituenten darstellen, ausgewählt aus der Gruppe α,
oder R₁₂ und R₁₃ zusammen mit einem zur Kopplungsstelle meta-ständigen Rest R₁₁ einen annellierten, unsubstituierten, mono- oder disubstituierten Benzo-, Naphtho- oder Pyridoring darstellen, dessen Substituenten aus der Gruppe α ausgewählt sein können, wobei dies bei einer Verbrückung mittels der Gruppen -O-CR₁₂R₁₃- bzw. -S-CR₁₂R₁₃- nur möglich ist, wenn CR₁₂R₁₃ direkt an den Phenylring, der R₁₁ trägt, gebunden ist, oder
im Falle einer Kopplung der vorstehenden Einheit (A) über R₇ und einer Verbrückung über R₆ die Reste R₁₂ und R₁₃ zusammen mit R₅ bzw. im Falle einer Kopplung der vorstehenden Einheit (A) über R₈ und einer Verbrückung über R₇ die Reste R₁₂ und R₁₃ zusammen mit R₆ bzw. im Falle einer Kopplung der vorstehenden Einheit (A) über R₆ und einer Verbrückung über R₇ die Reste R₁₂ und R₁₃ zusammen mit R₈ einen annellierten, unsubstituierten, mono- oder disubstituierten Benzo-, Naphtho- oder Pyridoring darstellen, dessen Substituenten aus der Gruppe α ausgewählt sein können, oder
R₁₄ und R₁₅ einen annellierten, unsubstituierten, mono- oder disubstituierten Benzo-, Naphtho- oder Pyridoring darstellen, dessen Substituenten aus der Gruppe α ausgewählt sein können;
m 0, 1, 2 oder 3 ist und
B und B' unabhängig voneinander aus einer der folgenden Gruppen a), b) oder c) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist;
wobei die Substituenten der Aryl- oder Heteroarylreste in a) und b) solche sind, ausgewählt aus der vorstehend definierten Gruppe α oder der Gruppe χ, bestehend aus Hydroxy, am Phenylring un-, mono- oder disubstituiertem 2-Phenylethenyl, am Phenylring un-, mono- oder disubstituiertem (Phenylimino)methylen, am Phenylring un-, mono- oder disubstituiertem (Phenylmethylen)imino, Amino, Mono-(C₁ - C₆)-alkylamino, Di-(C₁ - C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁ - C₆)-Alkyl, (C₁ - C₆)-Alkoxy, Brom, Chlor oder Fluor ausgewählt sein können;
oder wobei zwei direkt benachbarte Substituenten eine Y-(CX₂)ₚ-Z-Gruppierung darstellen, wobei p = 1, 2 oder 3 ist, X Wasserstoff, CH₃ oder C₆H₅ sein kann und Y und Z unabhängig voneinander Sauerstoff, Schwefel, N-(C₁ - C₆)-Alkyl, N-C₆H₅, CH₂, C(CH₃)₂ oder C(C₆H₅)₂ sein können, wobei zwei oder mehrere benachbarte Kohlenstoffatome dieser Y-(CX₂)ₚ-Z-Gruppierung jeweils unabhängig voneinander auch Teil eines daran annellierten Benzo-Ringsystems sein können, welches jeweils wiederum einen oder mehrere Substituenten, ausgewählt aus der Gruppe α oder der Gruppe χ, aufweisen kann;
oder
c) B und B' zusammen mit dem benachbarten Kohlenstoffatom des Pyran-Ringes einen un-, mono- oder disubstituierten 9,10-Dihydroanthracen-, Fluoren-, Thioxanthen-, Xanthen-, Benzo[b]fluoren-, 5H-Dibenzo[a,d]cyclohepten oder Dibenzosuberon-Rest oder einen gesättigten Kohlenwasserstoffrest, der (C₃ - C₁₂)-spiro-monozyklisch, (C₇ - C₁₂)-spiro-bizyklisch bzw. (C₇ - C₁₂)-spiro-trizyklisch ist, bilden, wobei die Substituenten der ungesättigten Cyclen unabhängig voneinander aus der Gruppe α oder der Gruppe χ ausgewählt sein können.

2. Photochrome Benzopyrano-benzopyrane gemäß Anspruch 1, welche die nachfolgende allgemeine Formel (II) aufweisen: 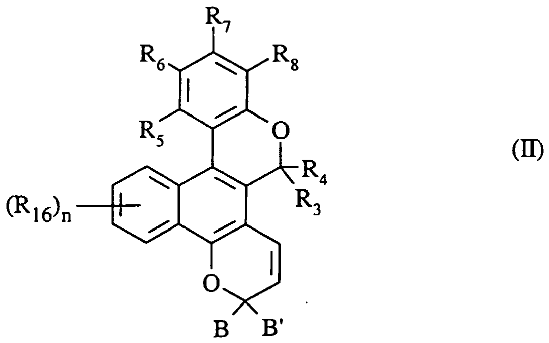 worin B, B', R₃, R₄, R₅, R₆, R₇ und R₈ wie vorgenannt definiert sind, R₁₆ aus der Gruppe α ausgewählt ist und n 0, 1, 2, 3 oder 4 ist.

3. Photochrome Benzopyrano-benzopyrane nach Anspruch 1 oder 2, wobei die Reste R₃ und R₄ jeweils unabhängig voneinander aus der Gruppe α ausgewählt.

4. Photochrome Benzopyrano-benzopyrane nach einem der Ansprüche 1 bis 3, wobei B und B' unabhängig voneinander aus der Gruppe a) ausgewählt sind.

5. Photochrome Benzopyrano-benzopyrane nach einem der Ansprüche 1 bis 4, welche die nachfolgenden allgemeinen Formeln (III) bzw. (IV) aufweisen: 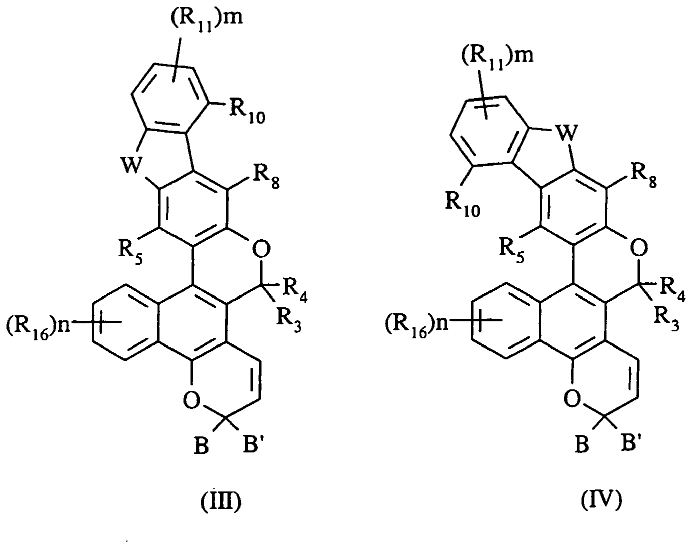 worin B, B', R₃, R₄, R₅, R₈, R₁₀ und R₁₆ wie vorgenannt definiert sind und W die vorstehend angeführte Verbrückung symbolisiert

6. Photochrome Benzopyrano-benzopyrane nach einem der Ansprüche 1 bis 4, welche die nachfolgenden allgemeinen Formeln (V), (VI) bzw. (VII) aufweisen: 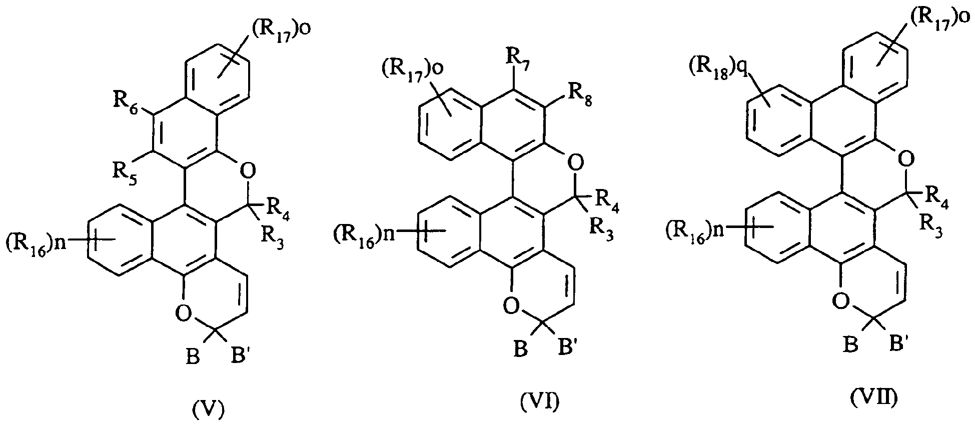 worin B, B', R₃, R₄, R₅, R₆, R₇, R₈, R₁₆ und n wie vorgenannt definiert sind, R₁₇ sowie R₁₈ aus der Gruppe α ausgewählt sind und o und q unabhängig voneinander 0, 1, 2, 3 oder 4 sind.

7. Verwendung der photochromen Benzopyrano-benzopyrane nach einem der Ansprüche 1 bis 6 in und auf Kunststoffmaterialien.

8. Verwendung nach Anspruch 7, wobei das Kunststoffmaterial eine ophthalmische Linse ist.

## Claims

1. Photochromic benzopyrano-benzopyrans prepared with further annelation with the general formula (I) 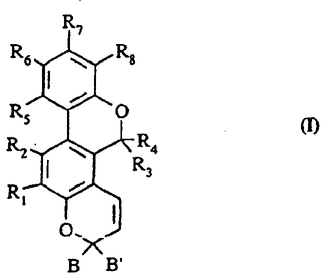 wherein at least one of the residues R₆, R₇ or R₈ represents the following unit (A), 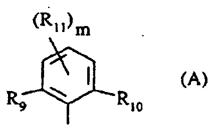 on condition that R₉ or R₁₀ together with a residue R₅, R₆, R₇ or R₈ that is ortho to the coupling point forms a bridge, or in the case of a coupling of the aforementioned unit (A) by means of R₆, R₉ and R₁₀ respectively together with both residues R₅ and R₇ ortho to the coupling point, or in the case of a coupling of the aforementioned unit (A) by means of R₇ with both residues R₆ and R₈ ortho to the coupling point form two bridges, wherein the bridging by means of residues R₉ or R₁₀ is respectively such a one selected from the group consisting of -CR₁₂R₁₃-, -O-, -S-, -N(Ph)-, -N(C₁-C₆ alkyl)-, -O-CR₁₂R₁₃-, S-CR₁₂R₁₃-, -CR₁₂R₁₃-CR₁₂R₁₃-, -CR₁₄=CR₁₅- or -CR₁₅=N⁻,
or
wherein residue R₅ with residue R₆ or residue R₆ with residue R₇ or residue R₇ with residue R₈ or residue R₅ with residue R₆ and at the same time residue R₇ with residue R₈ represent an un-, mono- or disubstituted benzo-, pyrido-, furo- or thieno-ring, wherein the substituents can respectively be selected from the group α consisting of a hydrogen atom, a (C₁-C₆) alkyl residue, a (C₁-C₆) thioalkyl residue, a (C₃-C₇) cycloalkyl residue that can have one or more heteroatoms such as O or S, for example, a (C₁-C₆) alkoxy residue, a hydroxy group or trifluoromethyl group, bromium, chlorine, fluorine, an un-, mono- or disubstituted phenyl-, phenoxy-, benzyl-, benzyloxy-, naphthyl- or naphthoxy residue, wherein the substituents can in turn be selected from group α;
and wherein
residues R₁ and R₂ respectively independently of one another represent a substituent selected from the above group α; or
residues R₁ and R₂ form an -A-(CH₂)ₖ-D- group or -A-(C(CH₃)₂)ₖ-D- group bonded to the aromatic ring where k = 1 or 2, wherein A and D independently of one another are selected from oxygen, sulphur, CH₂, C(CH₃)₂ or C(C₆(H₅)₂, and wherein a benzo-ring can in turn be annelated to this -A-(CH₂)ₖ-D- group; or
residues R₁ and R₂ represent an unsubstituted, mono- or disubstituted benzo- or pyrido-ring, the substituents of which can be selected from group α;
residues R₃ and R₄ are either selected from group α respectively independently of one another, or residues R₃ and R₄ together form with inclusion of the spiro-carbon atom a 3- to 8-member carbo- or heteromonocyclic spiro-ring, which possibly bears one or more substituents from group α, and to which one to three aromatic or heteroaromatic ring systems can be annelated, wherein the ring system is selected independently of one another from group β consisting of benzene, naphthaline, phenanthrene, pyridine, quinoline, furan, thiophene, pyrrole, benzofuran, benzothiophene, indole and carbazole, which can in turn be substituted with one or more substituents from group α, wherein two adjacent annelated ring systems can also be linked to one another by an ortho,ortho' bridge, or residues R₃ and R₄ together form with the inclusion of the spiro-carbon atom a 7- to 12-member carbo-bicyclic spiro-ring or a 7- to 12-member carbo-tricyclic spiro-ring, which respectively can possibly bear one or more substituents from group α,
residues R₅, R₆, R₇, R₈, R₉ and R₁₀, so long as they do not form a bridge, and also residue R₁₁ respectively independently of one another represent a substituent selected from group α or
in the case of a coupling of the above unit (A) by means of R₈, R₅ and R₆ represent an annelated, unsubstituted, mono- or disubstituted benzo-, naphtho- or pyrido-ring, the substituents of which can be selected from group α;
or two residues R₁₁ ortho to one another form an -A-(CH₂)ₖ-D- group or -A-(C(CH₃)₂)ₖ-D- group bonded to the aromatic ring where k = 1 or 2, as defined above, or two residues R₁₁ ortho to one another represent an unsubstituted, mono- or disubstituted benzo- or pyrido-ring, the substituents of which can be selected from group α;
residues R₁₂, R₁₃, R₁₄ and R₁₅ respectively independently of one another represent a substituents selected from group α.
or R₁₂ and R₁₃ together with a residue R₁₁ meta to the coupling point represent an annelated, unsubstituted, mono- or disubstituted benzo-, naphtho- or pyrido-ring, the substituents of which can be selected from group α, wherein this is only possible in the case of a bridging by means of groups -O-CR₁₂R₁₃- or -S-CR₁₂R₁₃- when CR₁₂R₁₃ is bonded directly to the phenyl ring that bears R₁₁, or
in the case of a coupling of the above unit (A) by means of R₇ and a bridging by means of R₆, residues R₁₂ and R₁₃ together with R₅, or in the case of a coupling of the above unit (A) by means of R₈ and a bridging by means of R₇, residues R₁₂ and R₁₃ together with R₆, or in the case of a coupling of the above unit (A) by means of R₆ and a bridging by means of R₇, residues R₁₂ and R₁₃ together with R₈ represent an annelated, unsubstituted, mono- or dissubstituted benzo-, naphtho- or pyrido-ring, the substituents of which can be selected from group α, or
R₁₄ and R₁₅ represent an annelated, unsubstituted, mono- or dissubstituted benzo-, naphtho- or pyrido-ring, the substituents of which can be selected from group α;
m is 0, 1, 2 or 3 and
B and B', independently of one another are selected from the following groups a), b) or c), wherein
a) are mono-, di- and trisubstituted aryl residues, wherein aryl residue is phenyl, naphthyl or phenanthryl;
b) are unsubtituted, mono- and disubstituted heteroaryl residues, wherein the heteroaryl residue is pyridyl, furanyl, benzofuranyl, thienyl, benzothienyl, 1, 2, 3, 4-tetrahydrocarbazolyl or julolidinyl;
wherein the substituents of aryl or heteroaryl residues in a) and b) are those selected from the above-defined group α or group χ consisting of hydroxy, 2-phenylethenyl un-, mono- or disubstituted on the phenyl ring, (phenylimino)methylene un-, mono- or disubstituted on the phenyl ring, (phenylmethylene) imino un-, mono- or disubstituted on the phenyl ring, amino, mono-(C₁-C₆) alkyl amino, Di-(C₁-C₆) alkyl amino, mono- and diphenylamino un-, mono- or disubstituted on the phenyl ring, piperidinyl, N-substituted piperazinyl, pyrrolidinyl, imidazolidinyl, pyrazolidinyl, indolinyl, morpholinyl, 2,6-dimethylmorpholinyl, thiomorpholinyl, azacycloheptyl, azacyclooctyl, un-, mono- or disubstituted phenothiazinyl, un-, mono- or disubstituted phenoxazinyl, un-, mono- or disubstituted 1,2, 3, 4-tetrahydroquinolinyl, un-, mono- or disubstituted 2, 3-dihydro-1,4-benzoxazinyl, un-, mono- or disubstituted1,2,3,4-tetrahydroisoquinolinyl, un-, mono- or disubstituted phenazinyl, un-, mono- or disubstituted carbazolyl, un-, mono- or disubstituted1, 2, 3, 4-tetrahydrocarbazolyl and un-, mono- or disubstituted10,11-dihydrodibenz[b,f]azepinyl, wherein the substituent or substituents, independently or one another, in turn can be selected from (C₁-C₆) alkyl, (C₁-C₆) alkoxy, bromine, chlorine or fluorine;
or wherein two directly adjacent substituents represent a Y-(CX₂)ₚ-Z grouping, wherein p = 1, 2 or 3, X can be hydrogen, CH₃ or C₆H₅ and Y and Z, independently of one another can be oxygen, sulphur, N-(C₁-C₆) alkyl, N-C₆H₅, CH₂, C(CH₃)₂ or C(C₆H₅)₂, wherein two or more adjacent carbon atoms of this Y-(CX₂)ₚ-Z grouping, respectively independently of one another, can also be a part of a benzo-ring system annelated thereto, which in turn can respectively have one or more substituents selected from group α or group χ; or
c) B and B' together with the adjacent carbon atom of the pyran ring form an un-, mono or disubstituted 9,10-dihydroanthracene, fluorene, thioxanthene, xanthene, benzo[b]fluorene, 5H-dibenzo[a,d]cycloheptene or dibenzosuberone residue or a saturated hydrocarbon residue, which is (C₃-C₁₂) spiro-monocyclic, (C₇-C₁₂) spiro-bicyclic or (C₇-C₁₂) spiro-tricyclic, wherein the substituents of the unsaturated cycles can be selected independently of one another from group α or group χ.

2. Photochromic benzopyrano-benzopyrans according to claim 1, which have the following general formula (II): 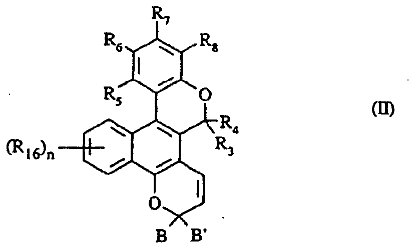 wherein B, B', R₃, R₄, R₅, R₆, R₇ and R₈ are defined as indicated above, R₁₆ is selected from group α and n is 0, 1, 2, 3 or 4.

3. Photochromic benzopyrano-benzopyrans according to claim 1 or 2, wherein residues R₃ and R₄ are respectively selected independently of one another from group α.

4. Photochromic benzopyrano-benzopyrans according to one of claims 1 to 3, wherein B and B' are respectively selected independently of one another from group a).

5. Photochromic benzopyrano-benzopyrans according to one of claims 1 to 4, which have the following general formulae (III) or (IV): 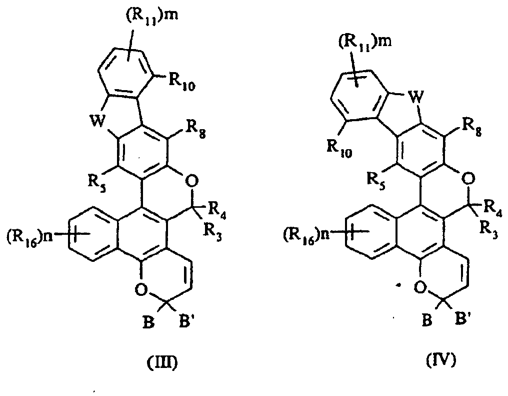 wherein B, B', R₃, R₄, R₅, R₈, R₁₀ and R₁₆ are defined as indicated above and W symbolises the bridging indicated above.

6. Photochromic benzopyrano-benzopyrans according to one of claims 1 to 4, which have the following general formulae (V), (VI) or (VII): 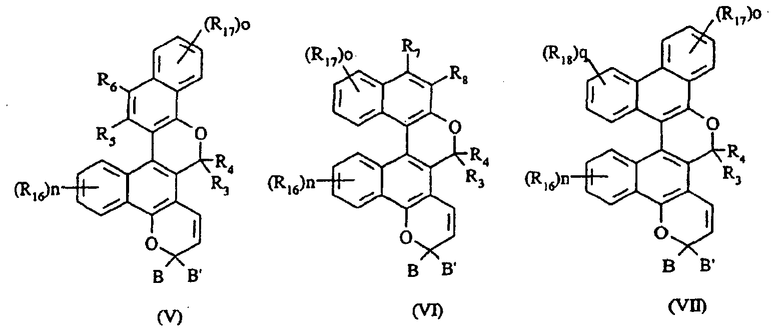 wherein B, B', R₃, R₄, R₅, R₆, R₇, R₈, R₁₆ and n are defined as indicated above, R₁₇ and also R₁₈ are selected from group α and o and q, independently of one another, are 0, 1, 2, 3 or 4.

7. Use of the photochromic benzopyrano-benzopyrans according to one of claims 1 to 6 in and on plastic materials.

8. Use according to claim 7, wherein the plastic material is an ophthalmic lens.

## Revendications

1. Benzopyrano-benzopyranes photochromes avec annélation supplémentaire selon la formule générale (I) mis à disposition: 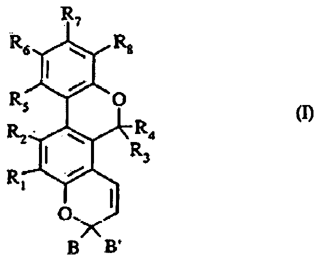 où au moins un des radicaux R₆, R₇ ou R₈ présente l'unité (A) suivante, 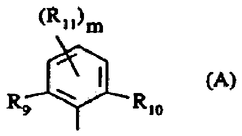 sous réserve que R₉ ou R₁₀, en combinaison avec un radical R₅, P₆, R₇ ou R₈ en position ortho par rapport au site de liaison forment un pontage, ou R₉ et R₁₀, dans le cas d'une liaison de l'unité (A) ci-dessus par l'intermédiaire de R₆, forment deux pontages respectivement en combinaison avec les deux radicaux R₅ et R₇ en position ortho par rapport au site de liaison, ou bien dans le cas d'une liaison de l'unité (A) ci-dessus par l'intermédiaire de R₇ forment deux pontages avec les deux radicaux R₆ et R₈ en position ortho par rapport au site de liaison, le pontage par l'intermédiaire des radicaux R₉ ou R₁₀ étant respectivement sélectionné parmi le groupe constitué de -CR₁₂R₁₃-, -O-, -S-, - N(Ph)-, -N(alkyle en C₁-C₆)-, -O-CR₁₂R₁₃-, -S-CR₁₂R₁₃-, -CR₁₂R₁₃-CR₁₂R₁₃-, - CR₁₄=CR₁₅- ou -CR₁₅=N-,
ou
dans laquelle le radical R₅ avec le radical R₆ ou le radical R₆ avec le radical R₇ ou le radical R₇ avec le radical R₈ ou le radical R₅ avec le radical R₆ et simultanément le radical R₇ avec le radical R₈ présentent un cycle benzo, pyrido, furo ou thiéno non substitué, monosubstitué ou disubstitué, les substituants pouvant être choisis respectivement parmi le groupe α constitué d'un atome d'hydrogène, d'un radical alkyle en C₁-C₆, d'un radical thioalkyle en C₁-C₆, d'un radical cycloalkyle en C₃-C₇, qui peut présenter un ou plusieurs hétéroatomes comme par exemple O ou S, d'un radical alcoxy en C₁-C₆, d'un groupe hydroxy, d'un groupe trifluorométhyle, d'un brome, d'un chlore, d'un fluor, d'un radical phényl, phénoxy, benzyle, benzyloxy, naphtyle ou naphtoxy non substitué, monosubstitué ou disubstitué, les substituants pouvant à nouveau être choisis parmi le groupe α ;
et dans laquelle
les radicaux R₁ et R₂ présentent respectivement indépendamment l'un de l'autre un substituant choisi parmi le groupe α ci-dessus ; ou
les radicaux R₁ et R₂ forment un groupe -A-(CH₂)ₖ-D- ou un groupe -A-(C(CH₃)₂)ₖ-D- lié au cycle aromatique avec k = 1 ou 2 ; A et D étant choisis indépendamment l'un de l'autre parmi oxygène, soufre, CH₂, C(CH₃)₂ ou C(C₆H₅)₂, et un cycle benzo pouvant à nouveau être annélé au niveau dudit groupe -A-(CH₂)ₖ-D- ; ou
les radicaux R₁ et R₂ présentent un cycle benzo ou pyrido non substitué, monosubstitué ou disubstitué, dont les substituants peuvent être choisis parmi le groupe α ;
les radicaux R₃ et R₄ sont choisis respectivement indépendamment l'un de l'autre parmi le groupe α ou bien les radicaux R₃ et R₄ forment ensemble et en incluant l'atome de carbone spiranique un cycle spiranique carbomonocyclique ou hétéromonocyclique triangulaire ou octogonal, qui porte éventuellement un ou plusieurs substituants issus du groupe α, au niveau desquels un à trois systèmes cycliques aromatiques ou hétéroaromatiques peuvent être annélés, le système cyclique étant choisi indépendamment des autres parmi le groupe β constitué de benzène, naphtaline, phénanthrène, pyridine, quinoline, furane, thiophène, pyrrole, benzofurane, benzothiophène, indole et carbazole, qui peuvent être à nouveau substitués avec un ou plusieurs substituants issus du groupe α, deux systèmes cycliques annélés voisins pouvant aussi être reliés l'un à l'autre par un pont ortho, ortho', ou bien les radicaux R₃ et R₄ forment ensemble et en incluant l'atome de carbone spiranique un cycle spiranique carbobicyclique heptagonal à dodécagonal ou un cycle spiranique carbotricyclique heptagonal à dodécagonal, qui peuvent respectivement porter éventuellement un ou plusieurs substituants issus du groupe α,
les radicaux R₅, R₆, R₇, R₈, R₉ et R₁₀, dans la mesure où ils ne forment pas un pontage, ainsi que le radical R₁₁ présentent respectivement indépendamment les uns des autres un substituant, choisi parmi le groupe α, ou
R₅ et R₆, dans le cas d'une liaison de l'unité (A) ci-dessus par l'intermédiaire de R₈, présentent un cycle benzo, naphto ou pyrido annélé non substitué, monosubstitué ou disubstitué, dont les substituants peuvent être choisis parmi le groupe α ;
ou deux radicaux R₁₁ en position ortho l'un par rapport à l'autre forment un groupe -A-(CH₂)ₖ-D- ou -A-(C(CH₃)₂)ₖ-D- lié au cycle aromatique avec k = 1 ou 2, comme défini ci-dessus, ou deux radicaux R₁₁ en position ortho l'un par rapport à l'autre présentent un cycle benzo ou pyrido non substitué, monosubstitué ou disubstitué, dont les substituants peuvent être choisis parmi le groupe α ;
les radicaux R₁₂, R₁₃, R₁₄ et R₁₅ présentent respectivement indépendamment les uns des autres un substituant choisi parmi le groupe α,
ou R₁₂ et R₁₃ présentent en commun avec un radical R₁₁ en position méta par rapport au site de liaison un cycle benzo, naphto ou pyrido annélé non substitué, monosubstitué ou disubstitué, dont les substituants peuvent être choisis parmi le groupe α, ceci, dans le cas d'un pontage faisant intervenir les groupes -O-CR₁₂R₁₃- ou -S-CR₁₂R₁₃-, n'étant possible que si CR₁₂R₁₃ est lié directement au cycle phényle qui porte R₁₁, ou
les radicaux R₁₂ et R₁₃ en combinaison avec R₅, dans le cas d'une liaison de l'unité (A) ci-dessus par l'intermédiaire de R₇ et d'un pontage par l'intermédiaire de R₆, ou bien les radicaux R₁₂ et R₁₃ en combinaison avec R₆, dans le cas d'une liaison de l'unité (A) ci-dessus par l'intermédiaire de R₈ et d'un pontage par l'intermédiaire de R₇, ou bien les radicaux R₁₂ et R₁₃ en combinaison avec R₈, dans le cas d'une liaison de l'unité (A) ci-dessus par l'intermédiaire de R₆ et d'un pontage par l'intermédiaire de R₇, présentent un cycle benzo, naphto ou pyrido annélé non substitué, monosubstitué ou disubstitué, dont les substituants peuvent être choisis parmi le groupe α, ou
R₁₄ et R₁₅ présentent un cycle benzo, naphto ou pyrido annélé non substitué, monosubstitué ou disubstitué, dont les substituants peuvent être choisis parmi le groupe α ;
m est égal à 0, 1, 2 ou 3 et
B et B' sont choisis indépendamment l'un de l'autre parmi l'un des groupes a), b) ou c) ci-dessous, dans lesquels
a) représente des radicaux aryle monosubstitués, disubstitués et trisubstitués, le radical aryle étant un groupe phényle, naphtyle, ou phénanthryle ;
b) représente des radicaux hétéroaryle non substitués, monosubstitués et disubstitués, le radical hétéroaryle étant un groupe pyridyle, furanyle, benzofuranyle, thiényle, benzothiényle, 1,2,3,4-tétrahydrocarbazolyle ou julolidinyle ;
les substituants des radicaux aryle ou hétéroaryle dans a) et b) étant choisis parmi le groupe α défini ci-dessous ou le groupe χ constitué de hydroxy, 2-phényléthényle non substitué, monosubstitué ou disubstitué au niveau du cycle phényle, (phénylimino)méthylène non substitué, monosubstitué ou disubstitué au niveau du cycle phényle, (phénylméthylène)imino non substitué, monosubstitué ou disubstitué au niveau du cycle phényle, amino, mono-alkylamino en C₁-C₆, di-alkylamino en C₁-C₆, monophénylamino et diphénylamino non substitué, monosubstitué ou disubstitué au niveau du cycle phényle, pipéridinyle, pipérazinyle substitué en N, pyrrolidinyle, imidazolidinyle, pyrazolidinyle, indolinyle, morpholinyle, 2,6-diméthylmorpholinyle, thiomorpholinyle, azacycloheptyle, azacyclooctyle, phénothiazinyle non substitué, monosubstitué ou disubstitué, phénoxazinyle non substitué, monosubstitué ou disubstitué, 1,2,3,4-tétrahydroquinolinyle non substitué, monosubstitué ou disubstitué, 2,3-dihydro-1,4-benzoxazinyle non substitué, monosubstitué ou disubstitué, 1,2,3,4,-tétrahydroisoquinolinyle non substitué, monosubstitué ou disubstitué, phénazinyle non substitué, monosubstitué ou disubstitué, carbazolyle non substitué, monosubstitué ou disubstitué, 1,2,3,4-tétrahydrocarbazolyle non substitué, monosubstitué ou disubstitué et 10,11-dihydrodibenzo[b,f]azépinyle non substitué, monosubstitué ou disubstitué, le ou les substituants pouvant être choisis indépendamment les uns des autres à nouveau parmi alkyle en C₁-C₆, alcoxy en C₁-C₆, brome, chlore et fluor ;
ou dans lesquels deux substituants immédiatement voisins présentent un groupement Y-(CX₂)ₚ-Z-, p étant égal à 1, 2 ou 3, X pouvant représenter un atome d'hydrogène, CH₃ ou C₆H₅ et Y et Z pouvant être indépendamment l'un de l'autre un atome d'oxygène, un atome de soufre, N-alkyle en C₁-C₆, N-C₆H₅, CH₂, C(CH₃)₂ ou C(C₆H₅)₂, deux ou plus de deux atomes de carbone voisins de ce groupement Y-(CX₂)ₚ-Z-pouvant respectivement indépendamment les uns des autres faire également partie d'un système cyclique benzo annélé qui peut présenter respectivement à nouveau un ou plusieurs substituants choisi parmi le groupe α ou le groupe χ;
ou
c) B et B' forment, en combinaison avec l'atome de carbone voisin du cycle pyrane, un radical 9,10-dihydroanthracène, fluorène, thioxanthène, xanthène, benzo[b]fluorène, 5H-dibenzo[a,d]cycloheptène ou dibenzosubérone non substitué, monosubstitué ou disubstitué ou un radical hydrocarbure saturé, qui est spiromonocyclique en C₃-C₁₂, spirobicyclique en C₇-C₁₂ ou bien spirotricyclique en C₇-C₁₂, les substituants des cycles insaturés pouvant indépendamment les uns des autres être choisis parmi le groupe α ou le groupe χ.

2. Benzopyrano-benzopyranes photochromes selon la revendication 1, qui présentent la formule générale (II) ci-dessous : 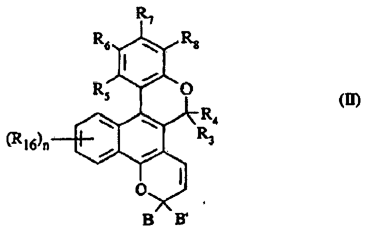 dans laquelle B, B', R₃, R₄, R₅, R₆, R₇ et R₈ sont définis tels que ci-dessus, R₁₆ est choisi parmi le groupe α et n représente 0, 1, 2, 3 ou 4.

3. Benzopyrano-benzopyranes photochromes selon la revendication 1 ou 2, où les radicaux R₃ et R₄ sont choisis respectivement indépendamment l'un de l'autre parmi le groupe α.

4. Benzopyrano-benzopyranes photochromes selon l'une quelconque des revendications 1 à 3, où B et B' sont choisis indépendamment l'un de l'autre parmi le groupe a).

5. Benzopyrano-benzopyranes photochromes selon l'une quelconque des revendications 1 à 4, qui présentent les formules générales (III) ou (IV) ci-dessous : 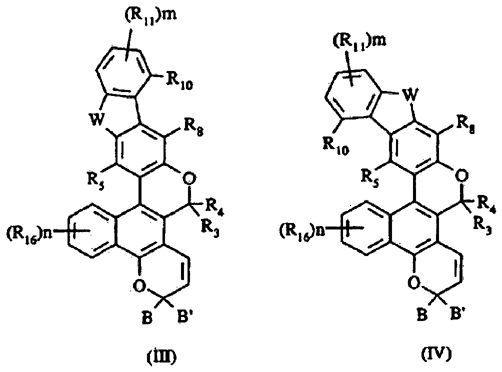 dans lesquelles B, B', R₃, R₄, R₅, R₈, R₁₀ et R₁₆ sont définis comme ci-dessus et W symbolise le pontage cité ci-dessus.

6. Benzopyrano-benzopyranes photochromes selon l'une quelconque des revendications 1 à 4, qui présentent les formules générales (V), (VI) ou (VII) ci-dessous : 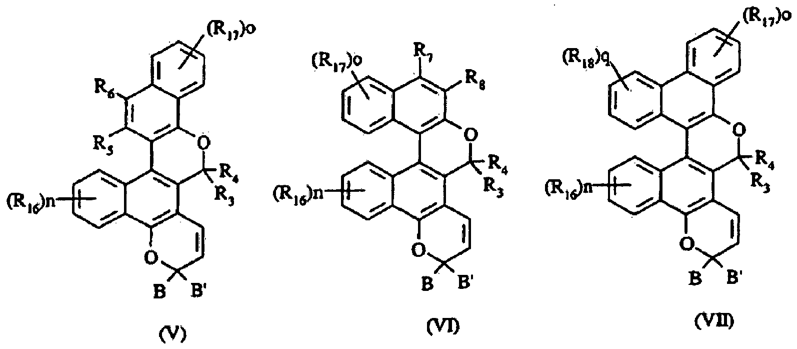 dans lesquelles B, B', R₃, R₄, R₅, R₆, R₇, R₈, R₁₆ et n sont définis comme ci-dessus, R₁₇ ainsi que R₁₈ sont choisis parmi le groupe α et o et q représentent indépendamment l'un de l'autre 0, 1, 2, 3 ou 4.

7. Utilisation des benzopyrano-benzopyranes photochromes selon l'une quelconque des revendications 1 à 6 dans et sur des matières plastiques.

8. Utilisation selon la revendication 7, où la matière plastique est une lentille ophtalmique.
